Europäisches Patentamt

European Patent Office

Office européen des brevets

(11) **EP 1 175 865 B1**

(19)

(12) **EUROPÄISCHE PATENTSCHRIFT**

(45) Veröffentlichungstag und Bekanntmachung des Hinweises auf die Patenterteilung:
**25.05.2005 Patentblatt 2005/21**

(51) Int Cl.$^7$: **A61B 5/0452**

(21) Anmeldenummer: **01117982.7**

(22) Anmeldetag: **25.07.2001**

(54) **Kardiologisches Geräteimplantat ausgestattet mit einem Auswerteverfahren zur Bestimmung der zeitlichen Stationarität gemessener kardiologischer Signale**

Cardiological implantable apparatus comprising an evaluation method for determining the temporal stationarity of measured cardiological signals

Appareil cardiologique implantable équipé avec un procédé d'évaluation pour la détermination de la stationarité temporelle de signaux cardiologique mesuré

(84) Benannte Vertragsstaaten:
**AT BE CH CY DE DK ES FI FR GB GR IE IT LI LU MC NL PT SE TR**

(30) Priorität: **28.07.2000 DE 10036842**

(43) Veröffentlichungstag der Anmeldung:
**30.01.2002 Patentblatt 2002/05**

(73) Patentinhaber: **BIOTRONIK Mess- und Therapiegeräte GmbH & Co Ingenieurbüro Berlin 12359 Berlin (DE)**

(72) Erfinder:
• **Meyer, Wolfgang**
 **91052 Erlangen (DE)**
• **Schaldach, Max, Prof. Dr.**
 **91054 Erlangen (DE)**

(74) Vertreter: **Hübner, Gerd, Dipl.-Phys. et al Rau, Schneck & Hübner Patentanwälte Königstrasse 2 90402 Nürnberg (DE)**

(56) Entgegenhaltungen:
**WO-A-97/33238      GB-A- 2 227 842 US-A- 5 842 997**

• **GLASS L ET AL: "TIME SERIES ANALYSIS OF COMPLEX DYNAMICS IN PHYSIOLOGY AND MEDICINE" MEDICAL PROGRESS THROUGH TECHNOLOGY, SPRINGER VERLAG. BERLIN, DE, Bd. 19, Nr. 3, 1993, Seiten 115-128, XP000423270 ISSN: 0047-6552**

**Beschreibung**

Die Erfindung betrifft ein kardiologisches Geräteimplantat, wie ein Defibrillator oder Herzschrittmacher, mit einem Betriebsprogramm, das ein Auswerteverfahren zur Bestimmung der zeitlichen Stationarität gemessener kardiologischer Signale implementiert.

Zum Verständnis der vorliegenden Erfindung ist deren Hintergrund vertieft aufzuzeigen. So ist das wichtigste Verfahren zur Gewinnung von Informationen über das Herz-Kreislauf-System die Registrierung des EKG durch an der Körperoberfläche eines Patienten angebrachte Elektroden. Alternativ dazu lassen sich durch eine geeignete Messanordnung über ein mit einer Elektrode verbundenes Implantat auch ein intrakardiales EKG oder ein den Kontraktionsablauf in völlig anderer Weise widerspiegelndes Signal, wie die intrakardiale Impedanz, aufzeichnen. In der Regel wird die Morphologie des Signalverlaufs ausgewertet, um pathologische Vorgänge zu diagnostizieren oder therapeutische Maßnahmen in geeigneter Weise einzuleiten.

Werden aus dem kontinuierlichen EKG-Signal beliebige diskrete Parameter extrahiert, wie z. B. der Abstand der R-Zacke des aktuellen Schlages vom Abstand der R-Zacke des vorhergehenden Schlages (RR-Intervalle), und die so erzeugte Folge von Werten in Abhängigkeit der fortlaufenden Nummer des Herzschlages aufgetragen, so erhält man eine Zeitreihe dieses kardialen Parameters.

Es wird nun davon ausgegangen, daß die Zeitreihe neben zufallsbedingten Einflüssen im wesentlichen das globale Regelungsverhalten des Herz-Kreislauf-Systems widerspiegelt, also in abstrakter Form Informationen über den Arbeitspunkt, die internen Übertragungsfunktionen zwischen Teilkomponenten oder Latenzzeiten des Informationstransfers zwischen Sensoren und Aktuatoren repräsentiert.

Durch Modellbildung aus Zeitreihen wird versucht, den Zustand des Herz-Kreislauf-Systems zu charakterisieren. Dies dient verschiedenen Zwecken, wie z.B. der Risikostratifizierung oder der Vorhersage potentiell tödlicher kardialer Ereignisse durch einen Defibrillator, oder der Eichung eines frequenzadaptiven Herzschrittmachers. Um eine korrekte Modellbildung zu gewährleisten, ist es erforderlich Zeitreihen auszuschließen, die durch zufällige Ereignisse beeinflußt werden, oder bei denen der Patient im Beobachtungszeitraum seinen physischen oder psychischen Belastungszustand ändert. Im ersten Fall repräsentiert ein aus der Messung erzeugtes deterministisches Modell nicht den tatsächlichen Zustand des Herz-Kreislauf-Systems. Im zweiten Fall ist aufgrund einer Drift oder eines sprunghaften Anstieges eines Parameters des Systemzustandes dieser Zustand überhaupt nicht definiert. Aus diesen Überlegungen wird ersichtlich, daß eine Auswahl bestimmter Abschnitte einer Zeitreihe hinsichtlich eines Stationaritätskriteriums eine Grundvoraussetzung für die Modellbildung und damit für die Charakterisierung von Zuständen darstellt.

Um in elektronischen Implantaten eingesetzt werden zu können, sind von einem Test auf Stationarität als weitere entscheidende Kriterien die Automatisierbarkeit und eine damit verbundene größtmögliche Einfachheit zu fordern, da in solchen Implantaten - zumindest nach dem derzeitigen Stand der Technik - die Rechenkapazität begrenzt ist. Dies liegt zum einen grundsätzlich daran, daß möglichst kompakte Rechenbausteine eingesetzt werden müssen, die zum anderen nur mit einer kapazitätsbegrenzten Energieversorgung arbeiten können. Wie im folgenden noch aufgezeigt wird, setzt die Erfindung an dieser Problematik an.

Zur Problematik der Stationaritätsbedingungen bei Messung einer beliebigen Zeitreihe sind bereits grundsätzlich gängige Tests bekannt, die auf einer aufwendigen statistischen Auswertung der Zeitreihen beruhen und als solche für eine Implementierung in kardiologischen Geräteimplantaten beim derzeitigen Stand der Prozessortechnik nicht geeignet erscheinen. Sie sind jedoch als solche im Zusammenhang mit der Erfindung zu erläutern, um den Einfluß von Störungen der Stationarität in einem Abschnitt einer Zeitreihe von Meßwerten auf die Auswertung selbst darlegen zu können. So lassen sich Störungen der Stationarität eines Messabschnitts in zufallsbedingte und dynamische Störungen einteilen. Zufallsbedingte Störungen umfassen im Herzen erzeugte Ereignisse, die nicht der Regelung des Kreislaufsystems unterliegen, wie ein temporärer AV-Block oder Extrasystolen, d. h. nicht durch Erregungsbildung im Sinusknoten bedingte Kontraktionen des Ventrikels. Zufallsbedingte Störungen ergeben sich darüber hinaus durch die Aufnahme von Reizen aus der Umgebung, wie plötzliche Geräusche, aber auch durch innere Vorgänge wie Motivationen, Änderungen der Aufmerksamkeit oder Träume. Dynamische Störungen ergeben sich z. B. im Tagesverlauf durch die mit der Ausübung verschiedener Tätigkeiten verbundenen, unterschiedlichen körperlichen und mentalen Belastungszustände eines Probanden, die mit wechselndem metabolischem Bedarf verbunden sind.

In physiologischen Experimenten lassen sich dynamische Störungen weitgehend vermeiden. Dies geschieht üblicherweise durch Präparation der Versuchsbedingungen, d. h. der Proband wird in reizarmer Umgebung bei gleichzeitiger Aufforderung zu passivem Verhalten untersucht. Der Vorgang der Untersuchung an sich löst jedoch vielfache psychische Vorgänge aus, die sich wenn überhaupt nur durch massive Verabreichung von Medikamenten beherrschen lassen. Diese Vorgehensweise steht jedoch der Untersuchung des Regelungsverhaltens des Herz-Kreislauf-Systems entgegen, da dieses durch die Medikamentierung beeinflußt wird.

Eine Präparation der Versuchsbedingungen ist somit im strengen Sinn nicht möglich, ein stationärer Abschnitt "a priori" somit nicht definierbar, was um so mehr aus der Sicht eines autonom arbeitenden Implantates gilt. In klinischen Versuchen werden stationäre Messintervalle meist "per Augenschein" und "a posteriori" festgelegt, d. h. der Experimentator wählt aus einer gegebenen Zeitreihe einen Abschnitt für die weitere Auswertung als stationär aus, in dem nach individuellen Maßstäben das Signal einen gleichmäßigeren Verlauf zu nehmen scheint, als in den übrigen Abschnitten. Die Nachteile dieser Vorgehensweise sind die Subjektivität dieses Auswahlverfahrens, die in erster Linie auf dem Fehlen quantitativer Kriterien beruht. Zudem kann ein Bereich als stationär definiert werden, der tatsächlich nicht stationär ist, womit die nachfolgenden Auswertungen fehlerhaft sind. Schließlich sind bei einer subjektiven Festlegung von angenommener Weise stationären Meßintervallen verschiedene Patienten nur bedingt vergleichbar. Auch sind derartige subjektive Auswahlverfahren naturgemäß nicht automatisierbar.

Um die zur Modellbildung und damit zu einer allgemeinen Diagnostik nötigen stationären Zustände zu identifizieren bzw. nichtstationäre Messabschnitte auszuschließen, ist daher ein Verfahren zu fordern, das eine "a posteriori"-Bestimmung der Stationarität ermöglicht, und gleichzeitig folgenden Kriterien gehorcht:

Das Verfahren muß objektiv sein, ein Kriterium für die grundsätzliche Existenz eines stationären Zustandes zur Verfügung stellen, sowie quantifizierbar, vergleichbar und automatisierbar sein.

Hinzu kommt, daß das Verfahren nicht auf bloßer Empirie beruhen sollte, sondern einen Bezug zu den mit nicht-stationärem Systemverhalten einhergehenden physiologischen Veränderungen herstellen sollte, um die Spezifität des Tests zu erhöhen.

Die einfachste Form eines statistischen Tests auf Stationarität beruht auf dem Vergleich der empirischen Verteilungen in zwei aufeinander folgenden Abschnitten. Dabei werden die statistischen Momente der Verteilungen bestimmt und mittels sogenannter "Zweistichproben-Tests" verglichen. Ergibt sich ein signifikanter Unterschied zwischen den Momenten der beiden Verteilungen, so wird die Annahme der Stationarität verworfen. Zwar handelt es sich dabei um ein sehr einfaches Verfahren, doch wird das Herz-Kreislauf-System dabei als rein stochastisches System interpretiert, d. h. als System mit einer unbegrenzten Anzahl von inneren Freiheitsgraden. Darüber hinaus wird das Kreislaufsystems in verschiedenen Zuständen durch identische Verteilungen repräsentiert.

Wird davon ausgegangen, daß abgesehen von externen und internen zufallsbedingten Störungen das Herz-Kreislauf-System deterministischen Entwicklungsbedingungen genügt, also der nachfolgende Schlag durch alle vorhergehenden exakt determiniert wird, so stellt die nichtlineare Dynamik verschiedene Stationaritätstests bereit. Da diese jedoch auf dem sogenannten Korrelationsintegral beruhen, sind sie sehr kompliziert, benötigen fundierte Kenntnis über eine Anzahl unbekannter Parameter und erfordern die Existenz vergleichsweise langer stationärer Abschnitte. Darüber hinaus stellt die Annahme eines streng deterministischen Systems als Grundlage der Regulation des Herz-Kreislauf-Systems sicherlich eine ebenso unrealistische Annahme dar, wie dessen vollständige Zufälligkeit. Da die große Zahl interner Freiheitsgrade nicht vollständig gekoppelt sein dürfte, ist auch bei kompletter Ausschaltung aller stochastischen Störungen ein internes Systemrauschen zu erwarten.

Realistischer ist die Annahme, daß im Herz-Kreislauf-System Korrelationen eine große Rolle spielen, diese jedoch auch im stationären Zustand von einem internen Rauschen überlagert werden. Für empirische Zwecke ist es nicht nötig, diese Anteile näher zu spezifizieren, und es genügt, ein Verfahren für einen Stationaritätstest zu finden, der beiden Faktoren Rechnung trägt. Eine Kombination stochastischen Verhaltens mit deterministischen Korrelationen erfaßt das im folgenden beschriebene Konzept der fraktionellen Brownschen Bewegung (fBm).

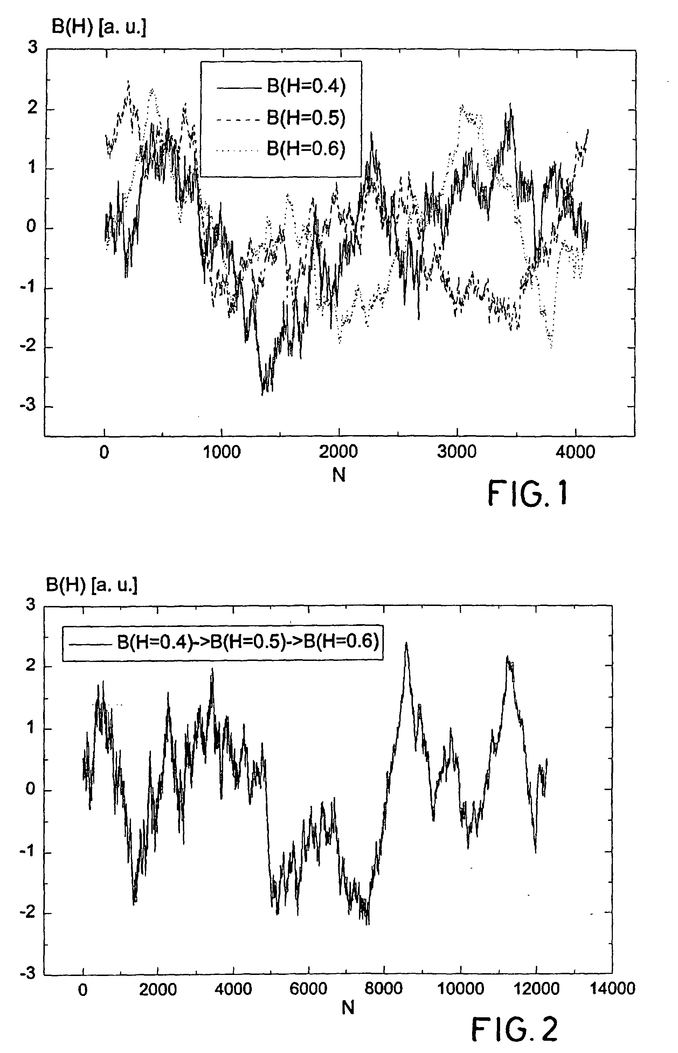

Wird der Verlauf einer kardialen Zeitreihe als die in der Zeit gerichtete Bewegung eines einzelnen Teilchens interpretiert, das sich in Richtung der y-Achse zu verschiedenen Orten begeben kann, so erhält man einen Random-Walk bzw. eine eindimensionale Brownsche Bewegung. Diese stimmt mit der gewöhnlichen stochastischen Brownschen Bewegung z. B. eines Teilchens überein, sofern der jeweilige Bewegungsschritt völlig unabhängig von allen vorherigen erfolgt. Hängt der jeweilige Bewegungsschritt jedoch teilweise von der Bewegung früherer Schritte ab, so spricht man von fraktioneller Brownscher Bewegung. Hierbei treten zwei grundsätzlich verschiedene Fälle auf. Der aktuelle Bewegungsschritt kann in Richtung des oder der früheren Schritte erfolgen, oder er kann diesen entgegengesetzt sein. Je stärker die eine oder andere Wahrscheinlichkeit ausgeprägt ist, desto stärker wird der Verlauf der Zeitreihe im Vergleich zur Brownschen Bewegung, bei der die Wahrscheinlichkeit für beide Richtungen identisch ist, davon geprägt. Die Art dieser Korrelation wird z. B. durch den Hurstschen Skalierungsexponenten H gemessen, der zwischen 0.0 und 1.0 liegen kann. Ein hoher Wert entspricht dabei einem sehr glatten Verlauf der Zeitreihe, da der aktuelle Schritt mit hoher Wahrscheinlichkeit in Richtung der vorherigen führt. Bei niedrigem Wert von H erscheint die Zeitreihe "aufgerauht". Dies wird anhand einer entsprechenden Darstellung von simulierten Zeitreihen im Ausführungsbeispiel noch näher erläutert (s. dort Fig. 1).

Bei der Überprüfung von Meßwert-Zeitreihen auf Stationarität wird nun im vorliegenden Zusammenhang davon ausgegangen, daß zwei Teilabschnitte einer Zeitreihe dann das System in identischen Zuständen repräsentieren, wenn ihr Hurstscher Skalierungsexponent den selben Wert aufweist. In diesem Fall wird angenommen, daß das System während der beiden Teilabschnitte stationär ist. Es muß ein Schwellenwert angegeben werden, unterhalb dessen ein Unterschied noch nicht signifikant angenommen werden kann. Dieser ist empirisch bzw. aus Simulationen zu bestimmen. Hierbei ist zu beachten, daß die Realisierung einer fraktionellen Brownschen Bewegung trotz der Korrelationen eine stochastische Komponente enthält, und zwei Zeitabschnitte niemals exakt und mit 100% Sensitivität unterschieden werden können.

Die darauf beruhenden Schwierigkeiten bei einer Unterscheidung verschiedener Abschnitte werden im folgenden in der Beschreibung des Ausführungsbeispiels anhand von Fig. 2 noch näher erläutert.

Der Hurstsche Skalierungsexponent ist nun zwar grundsätzlich für die Bewertung von Korrelationen und damit für einen Stationaritätstest von Zeitreihen geeignet, jedoch ist die Bestimmung dieses Exponenten mit den bekannten, gängigen Verfahren aufwendig, womit die Implementierung in einem kardiologischen Geräteimplantat nicht praktikabel ist. Zudem ist die exakte Quantifizierung des Hurstschen Skalierungsexponenten mit gängigen Verfahren insbesondere bei kürzeren Zeitreihen schwierig, wie sie vornehmlich im Zusammenhang mit kardiologischen Meßwerten in der Anwendung bei Geräteimplantaten relevant sind.

Ausgehend von der umfassend geschilderten Problematik des Standes der Technik liegt der Erfindung die Aufgabe zugrunde, ein kardiologisches Geräteimplantat mit einem darauf implementierten Auswerteverfahren anzugeben, mit dem auf einfache Weise und mit begrenzter Rechenkapazität die zeitliche Stationarität gemessener kardiologischer Signale bestimmbar ist.

Diese Aufgabe wird durch ein Geräteimplantat implementierend ein Auswerteverfahren mit folgenden Verfahrensschritten gelöst:

- Aufzeichnung der Werte eines kardiologischen Signals während einer bestimmten Meßdauer,
- Einteilung der so erhaltenen Zeitreihe von Signalwerten in einzelne Abschnitte,
- Abbilden aufeinanderfolgender Untermengen der einzelnen Signalwerte in einem Abschnitt auf zugeordnete Symbolreihen nach dem Kriterium, ob sich ein jeweiliger Signalwert gegenüber dem vorhergehenden Signalwert erhöht oder erniedrigt,
- Bestimmung der Häufigkeiten der verschiedenen Symbolreihen,
- Bestimmung eines das Häufigkeitsverhältnis von korrelierten zu antikorrelierten Symbolreihen innerhalb eines Abschnittes wiedergebenden Prüfparameters,
- Bestimmung des relativen Unterschiedes der Prüfparameter zweier benachbarter Abschnitte, und
- Vergleichen des relativen Unterschiedes zweier Prüfparameter mit einem vorgegebenen Schwellenwert als Kriterium für die Stationarität der Zeitreihe des aufgezeichneten physiologischen Signals.

Das vorstehende Verfahren ist schlagwortartig unter dem Begriff "Symbolische Dynamik" zu subsumieren. Dabei wird eine Zeitreihe auf eine Folge diskreter Symbole abgebildet, wobei es zur Komprimierung der in der Zeitreihe enthaltenen Information kommt. Da die interessierende Information bezüglich kardialer Zeitreihen aus Korrelationen und Antikorrelationen besteht, werden durch die hier verwendeten Symbole nur die jeweiligen Richtungen der beispielsweise das jeweilige RR-Intervall repräsentierenden "Bewegung" B(H) abgebildet. Nimmt B(H) - also der Signalwert - zu, so wird dem Schritt das Symbol "1" zugeordnet, nimmt B(H) ab, dann entspricht dem das Symbol "0". Das Abbilden der einzelnen Signalwerte B (H) der Zeitreihe entspricht bevorzugtermaßen also einer Abbildungsfunktion

$$B_n(H) = 1 \text{ für } B_{n+1}(H) - B_n(H) \geq 0$$

und

$$B_n(H) = 0 \text{ für } B_{n+1}(H) - B_n(H) < 0$$

auf Bitworte einer definierten Bitzahl mit den Elementen 0 und 1.

Um zu untersuchen, ob die Richtung häufiger geändert oder beibehalten wird, werden Bitworte mit vorzugsweise fünf Symbolen gebildet, also z. B. "01010", was einer ausgeprägten Antikorrelation entspricht. Die Häufigkeit von Worten, die einer überwiegenden Antikorrelation entsprechen, werden nun zur Häufigkeit der Worte in Beziehung gesetzt, die eine Korrelation repräsentieren. Durch dieses Verhältnis wird ein Prüfparameter bestimmt, dessen Wert für zwei Intervalle verglichen wird und so einen quantitativen Vergleichsmaßstab liefert.

Die Durchführung mit Hilfe eines anderen Verfahrens, daß stochastische Korrelationen mißt, erfolgt analog, mit dem Unterschied, daß der Prüfparameter an das jeweilige Verfahren anzupassen ist.

Zusammenfassend besteht der Unterschied zwischen symbolischer Dynamik und direkter Berechnung von H in der fehlenden Berücksichtigung langreichweitiger Korrelationen, die jedoch bei den kurzen zu untersuchenden Abschnitten physiologischer stationärer Zeitreihen weniger relevant sind. Auch wird die Eigenschaft der Selbstähnlichkeit, die eine fraktionelle Brownsche Bewegung definiert, nicht streng geprüft, was jedoch von untergeordnetem Interesse für den hier verfolgten praktischen Zweck ist. Ein Vorteil der symbolischen Dynamik liegt darin, daß Extrasystolen nicht überproportional stark aufgrund ihrer Höhe gewichtet werden. Auf eine entsprechende Filterung kann daher verzichtet werden. Aufgrund der Kurz-Lang-Sequenz bei Extrasystolen kann jedoch eine große Zahl von Extrasystolen zu einer Verschiebung der Korrelation in Richtung gegenläufiger Beziehungen zwischen den Einzelschritten der Bewegung führen. Eine weitere Anpassung der Wortlänge auf empirischer Grundlage erscheint denkbar.

Weiter Merkmale, Einzelheiten und Vorteile der Erfindung sind der nachfolgenden Beschreibung entnehmbar, in der ein Ausführungsbeispiel anhand der beigefügten Zeichnungen näher erläutert wird. Dabei zeigen:

Fig. 1   ein Kurvendiagramm dreier simulierter Meßwertreihen B (H) in Abhängigkeit von Meßpunkten N,

Fig. 2   ein Kurvenschaubild der drei Meßkurven B (H) gemäß Fig. 1 in aneinandergereihter Darstellung mit steigendem Hurstschen Skalierungsexponenten H in Abhängigkeit der Meßpunkte N,

Fig. 3   ein Flußdiagramm des erfindungsgemäßen Auswerteverfahrens,

Fig. 4   ein Auswertediagramm des Prüfparameters M in Abhängigkeit einer Abschnittsnummer n, und

Fig. 5   ein Auswertediagramm des relativen Unterschiedes $\Delta$ in Abhängigkeit der Abschnittsnummer n.

Fig. 1 zeigt eine simulierte Zeitreihe des Meßwertes B (H) in willkürlichen Einheiten (a.u.) in Abhängigkeit simulierter Meßpunkte N, deren Wertebereich sich von 0 bis 4000 erstreckt. Wie aus der Legende in dem Meßdiagramm gemäß Fig. 1 deutlich wird, stellen die Kurvenlinien Meßkurven mit unterschiedlichen Hurstschen Skalierungsexponenten H dar, wobei die durchgezogene Linie einen niedrigen Exponenten von H = 0.4, die strichlierte Linie einen mittleren Exponenten H = 0.5 und die punktierte Linie einen hohen Skalierungsexponenten H = 0.6 widerspiegelt. Wie bereits angesprochen, werden die Meßkurven mit steigendem Exponenten H glatter. Entsprechende Meßkurven ergeben sich beispielsweise beim Auftragen der jeweiligen Dauer des RR-Intervalls des Herzens über eine entsprechende Anzahl von Meßpunkten N.

Fig. 2 zeigt die "Hintereinanderschaltung" der drei in den Fig. 1 dargestellten Simulationskurven und verdeutlicht die Schwierigkeit der Unterscheidungen verschiedener Abschnitte einer Zeitreihe. Aus Fig. 2 läßt sich zwar intuitiv eine gewisse Glättung der Kurve mit steigendem N erahnen, was dem ansteigenden Hurstschen Skalierungsexponenten entspricht. Der Übergang zwischen den einzelnen Abschnitten ist jedoch nicht quantifizierbar. Bei Annahme einer Nicht-Stationarität aufgrund von großen Sprüngen des Meßwertes B (H) würde dieser sogar fehlerhaft bei N = 5000 diagnostiziert. Erst durch eine Berechnung des Skalierungsexponenten H selbst läßt sich den drei Abschnitten eine unterschiedliche Dynamik zuordnen.

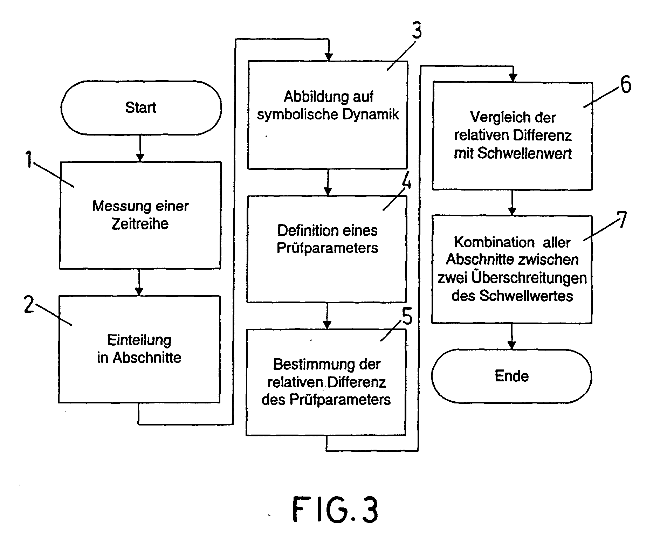

Unter Bezugnahme auf die Fig. 2 bis 5 wird nun das erfindungsgemäße Auswerteverfahren erläutert. Begonnen wird mit der Aufzeichnung der Werte eines kardiologischen Signals während einer bestimmten Meßdauer, womit beispielsweise eine Zeitreihe von RR-Intervallen gemessen wird (Schritt 1 in Fig. 3). Ein Diagramm der in Fig. 2 gezeigten Art repräsentiert diese Zeitreihe mit N = 12400 Meßpunkten. Das Auswerteverfahren hat nun zum Ziel, die Stationarität der Zeitreihe oder bestimmter Teilabschnitte davon zu verifizieren, damit diese Zeitreihe oder die Teilabschnitte einer weiteren sinnvollen Signalverarbeitung zugeführt werden können, damit daraus physiologisch verläßliche Schlußfolgerungen für die Steuerung des Implantates abgeleitet werden können.

Anschließend wird die Zeitreihe in Abschnitte identischer Länge unterteilt (Schritt 2 in Fig. 3). Die Länge eines einzelnen Abschnittes darf nicht zu groß gewählt werden, da so nicht-stationäre Intervalle innerhalb eines Abschnittes übersehen werden können. Sie darf nicht zu kurz gewählt werden, da sonst das Ergebnis für einen Prüfparameter nicht mit ausreichender Signifikanz bestimmt wird.

Der Prüfparameter wird nun abschnittsweise bestimmt. Er wird folgendermaßen mit Hilfe der symbolischen Dynamik definiert: Untermengen der einzelnen Signalwerte werden abgebildet auf eine Symbolfolge aus "0" und "1", je nachdem, ob der Signalwert B (H) ab- oder zunimmt (Schritt 3 in Fig. 3). Diese Abbildungsfunktion läßt sich wie folgt charakterisieren:

$$B_n(H) = 1 \text{ für } B_{n+1}(H) - B_n(H) >/= 0$$

und

$$B_n(H) = 0 \text{ für } B_{n+1}(H) - B_n(H) < 0$$

Die "Buchstaben" der Symbolfolge werden dann jeweils zu einem Wort aus fünf Zeichen zusammengefaßt, so daß sich Worte z. B. der Form "01010" oder "00000" ergeben. Die Häufigkeiten des Auftretens bestimmter Worte in dem Abschnitt, z. B. P("01010") oder P("00000") werden gezählt.

Die Summe von Worthäufigkeiten mit gleichgerichteter Korrelation ($\Sigma P_{korr}$), wie P("00000") + P("11111") wird in ein Verhältnis gesetzt mit der Summe von Worthäufigkeiten gegengerichteter Korrelation ($\Sigma P_{antikorr}$) wie P("10101") + P("01010").

Wird eine Einteilung der Zeitreihe aus Fig. 2 (Gesamtlänge 12400) in Abschnitte der Länge 1000 vorgenommen, und als Prüfparameter der Quotient

$$M = (\Sigma P_{korr})/(\Sigma P_{antikorr}) \text{ bzw.}$$

$$M = \frac{P(00001) + P(01111) + P(10000) + P(11110) + P(11111)}{P(10101) + P(00101) + P(01010) + P(01011) + P(10110) + P(01101) + P(01001) + P(11010) + P(10010) + P(10100)}$$

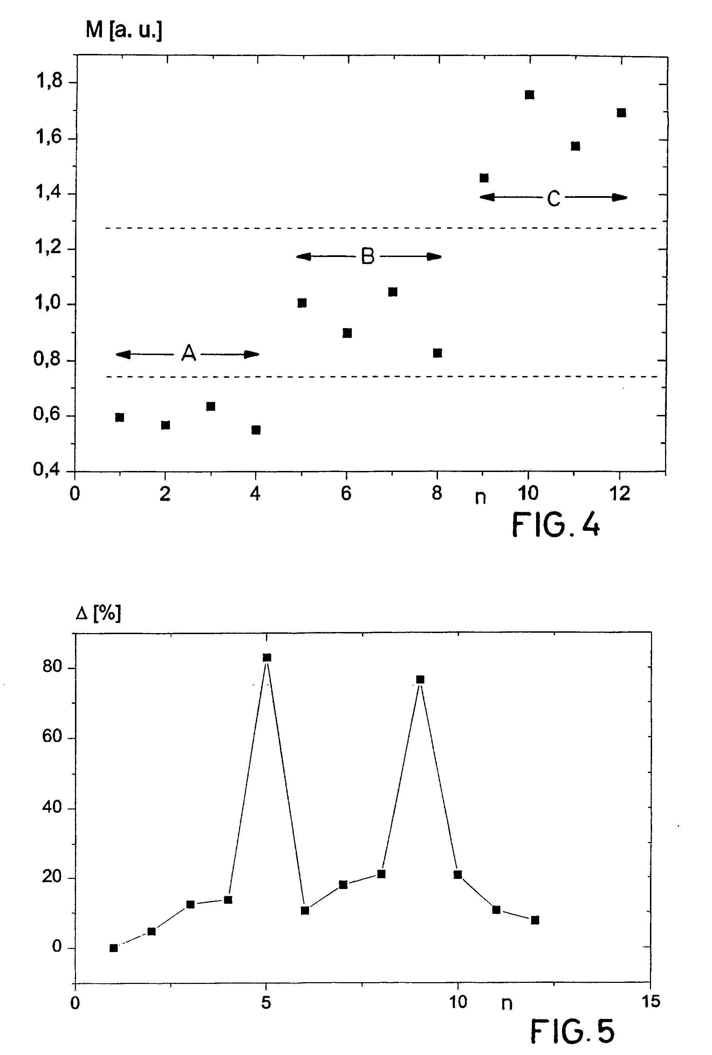

definiert (Schritt 4 in Fig. 3), wobei die Verwendung einzelner Worte wie die Abschnittslänge bei kardialen Zeitreihen empirisch zu bestimmen ist, so ergibt sich der in Fig. 4 dargestellte Zusammenhang. Die Ergebnisse der Prüfparameter M für die einzelnen Abschnitte der Zeitreihe aus Fig. 2 sind über der Nummer n des Abschnitts aufgetragen. Es wird deutlich, daß drei Abschnitte A, B und C (Fig. 4) in der Zeitreihe existieren, die untereinander disjunkte Mengen von Werten des Prüfparameters aufweisen. Hieraus läßt sich das weitere Vorgehen eines automatisierten Tests ableiten.

Nun wird abgeprüft, ob sich die Prüfparameter zweier benachbarter Abschnitte über ein gewisses Maß hinaus unterscheiden. Hierzu wird als Maßzahl der relative Unterschied $\Delta$ ermittelt (Schritt 5 in Fig. 3) als:

$$\Delta = \frac{M_{i+1} - M_i}{M_i} \times 100\%.$$

Für die Zeitreihe aus Fig. 2 ist die Maßzahl $\Delta$ über der Nummer des Abschnittspaares n in Fig. 5 dargestellt.

Aus Fig. 5 wird deutlich, daß es genügt, ein weiteres Schwellwertkriterium zu definieren, um zu entscheiden, ob Paare von Abschnitten gleichartige oder verschiedenartige Dynamik aufweisen. Dieser Schwellwert ist für kardiale Zeitreihen empirisch festzulegen. Für das gewählte Beispiel läßt sich leicht ein Schwellwert von z. B. 50% für die relative Differenz des Prüfparameters M angeben, unterhalb der ein Paar von Abschnitten als stationär gilt. Ein Paar von Abschnitten, für das die relative Differenz des Prüfparameters 50% überschreitet, wird damit verworfen (Schritt 6 in Fig. 3). Bezüglich Fig. 5 ist darauf hinzuweisen, daß sich die Abschnitte mit unterschiedlicher Dynamik lediglich um $\Delta H=0.1$ unterscheiden, so daß das Auswerteverfahren offensichtlich sehr sensitiv reagiert. Bei geringerer Sensitivität läßt sich analog Fig. 4 zusätzlich der Prüfparameter direkt auswerten.

Für die weitere Auswertung und Modellbildung der Zeitreihe in Fig. 2 ergibt sich aus dem in Fig. 5 dargestellten Verlauf der Maßzahl $\Delta$, daß jeweils die Abschnitte n = 1-4, n = 5-8 und n = 9-12 für weitere Auswertungen und die Modellbildung zusammengefaßt werden dürfen (Schritt 7 in Fig. 3). Eine Modellbildung darf jedoch keine Abschnitte gleichzeitig umfassen, die in verschiedenen der drei vorgefundenen Bereiche liegen.

Zu beachten ist darüber hinaus, daß die Stationarität für das jeweilige Abschnittspaar global gilt, d. h. es ist nicht ausgeschlossen, daß nicht-stationäre Bereiche innerhalb eines der Abschnitte auftreten, sofern die Abschnittslänge zu groß gewählt ist. Dem ist durch schrittweise Verkleinerung der Abschnittslänge vorzubeugen. Weitere Variationen des Verfahrens sind denkbar, wie z. B. eine Flexibilisierung der Abschnittsbreite oder gleitende Abschnitte.

**Patentansprüche**

1. Kardiologisches Geräteimplantat, insbesondere Defibrillator oder Herzschrittmacher, mit einem Betriebssteuerprogramm, das ein Auswerteverfahren zur Bestimmung der zeitlichen Stationarität vom Implantat gemessener kardiologischer Signale mit folgenden kennzeichnenden Verfahrensschritten implementiert:

   - Aufzeichnung der Werte eines kardiologischen Signals während einer bestimmten Meßdauer,
   - Einteilung der so erhaltenen Zeitreihe von Signalwerten in einzelne Abschnitte,
   - Abbilden aufeinanderfolgender Untermengen der einzelnen Signalwerte in einem Abschnitt auf zugeordnete Symbolreihen nach dem Kriterium, ob sich ein jeweiliger Signalwert gegenüber dem vorhergehenden Signalwert erhöht oder erniedrigt,
   - Bestimmung der Häufigkeiten der verschiedenen Symbolreihen,

- Bestimmung eines das Häufigkeitsverhältnis von korrelierten zu antikorrelierten Symbolreihen innerhalb eines Abschnittes wiedergebenden Prüfparameters,
- Bestimmung des relativen Unterschiedes der Prüfparameter zweier benachbarter Abschnitte, und
- Vergleichen des relativen Unterschiedes zweier Prüfparameter mit einem vorgegebenen Schwellenwert als Kriterium für die Stationarität der Zeitreihe des aufgezeichneten kardiologischen Signals.

2. Geräteimplantat nach Anspruch 1, **dadurch gekennzeichnet, daß** als kardiologisches Signal die jeweilige Dauer des RR-Intervalls des Herzens aufgezeichnet wird.

3. Geräteimplantat nach Anspruch 1 oder 2, **dadurch gekennzeichnet, daß** das Abbilden der einzelnen Signalwerte B(H) der Zeitreihe durch eine Abbildungsfunktion

$$B_n(H) = 1 \text{ für } B_{n+1}(H) - B_n(H) >/= 0$$

und

$$B_n(H) = 0 \text{ für } B_{n+1}(H) - B_n(H) < 0$$

auf Bitworte einer definierten Bitzahl mit den Symbol-Elementen 0 und 1 als jeweilige Symbolreihen erfolgt.

4. Geräteimplantat nach Anspruch 3, **dadurch gekennzeichnet, daß** die Bitworte jeweils fünf Symbol-Elemente enthalten.

5. Geräteimplantat nach Anspruch 3 oder 4, **dadurch gekennzeichnet, daß** als Prüfparameter (M) die Summe der Bitwort-Häufigkeiten ( $P_{korr}$) mit gleichgerichteten Korrelationen ins Verhältnis zur Summe der Bitwort-Häufigkeiten ($P_{antikorr}$) mit gegengerichteten Korrelationen nach der Beziehung

$$M = (\Sigma \, P_{korr})/(\Sigma \, P_{antikorr})$$

gesetzt wird.

6. Geräteimplantat nach Anspruch 5, **dadurch gekennzeichnet, daß** der relative Unterschied ($\Delta$) der Prüfparameter (M) zweier benachbarter Abschnitte nach der Beziehung

$$\Delta = (M_{i+1} - M_i)/M_i \times 100\%$$

bestimmt wird.

7. Geräteimplantat nach Anspruch 6, **dadurch gekennzeichnet, daß** der Schwellenwert für die Stationarität der Zeitreihe bei 50% liegt.

8. Geräteimplantat nach einem der Ansprüche 1 bis 7, **dadurch gekennzeichnet, daß** zusammenhängende Abschnitte, in denen das Stationaritätskriterium erfüllt ist, als Basis für eine weitere kardiologische Auswertung der aufgezeichneten kardiologischen Signale herangezogen werden.

**Claims**

1. An implantable cardiologic device, in particular a defibrillator or cardiac pacemaker, including an operation control program which implements an evaluation method for determination of time stationarity of cardiologic signals measured by the implant comprising the following **characterizing** steps:

   - recording the values of a physiological signal during a certain measuring period;
   - dividing the obtained time series of signal values into individual segments;

- mapping successive subsets of the individual signal values in a segment on associated symbol series according to the criterion of whether a respective signal value increases or decreases as compared to the preceding signal value;
- determining the frequencies of the varying symbol series;
- determining a testing parameter which reflects the frequency ratio, within a segment, of symbol series of varying correlation stages, in particular of correlated symbol series to anticorrelated symbol series;
- determining the relative difference of the testing parameters of two adjacent segments; and
- comparing the relative difference of two testing parameters with a given threshold as a criterion for the stationarity of the time series of the recorded cardiologic signal.

2. An implantable device according to claim 1, **characterized in that** the respective duration of the RR interval of the heart is recorded as a cardiological signal.

3. An implantable device according to claim 1 or 2, **characterized in that** the individual signal values B(H) of the time series are mapped as respective symbol series on bit words of a defined bit number comprising the symbol elements 0 and 1 by a function of representation

$$B_n(H) = 1 \text{ für } B_{n+1}(H) - B_n(H) \geq 0$$

and

$$B_n(H) = 0 \text{ für } B_{n+1}(H) - B_n(H) < 0.$$

4. An implantable device according to claim 3, **characterized in that** each bit word includes five symbol elements.

5. An implantable device according to claim 3 or 4, **characterized in that** the sum of bit word frequencies ($P_{korr}$) of equidirectional correlations is related to the sum of bit word frequencies ($P_{antikorr}$) of opposite correlations as a testing parameter (M) by the equation

$$M = (\Sigma P_{korr})/(\Sigma P_{antikorr}).$$

6. An implantable device according to claim 5, **characterized in that** the relative difference ($\Delta$) of the testing parameters (M) of two adjacent segments is determined by the relation

$$\Delta = (M_{i+1} - M_i)/M_i \times 100\%.$$

7. An implantable device according to claim 6, **characterized in that** the time series stationarity threshold is in the range of 50 %.

8. An implantable device according to one of claims 1 through 7, **characterized in that** coherent segments complying with the criterion of stationarity are used as a basis for further cardiologic evaluation of the recorded cardiologic signals.


**Revendications**

1. Appareil cardiologique implantable, en particulier défibrillateur ou stimulateur cardiaque, avec un programme de commande qui met en oeuvre un procédé d'évaluation de la stationnarité temporelle de signaux cardiologiques mesurés par l'implant avec les étapes de procédé caractéristiques suivantes :

- enregistrement des valeurs d'un signal cardiologique pendant une certaine période de mesure,
- division des séries temporelles des valeurs de signaux obtenues en différentes sections,
- représentation de sous-ensembles successifs de différentes valeurs de signaux dans une section sur des séries de symboles affectées selon le critère déterminant si une valeur de signal donnée augmente ou diminue par rapport à la valeur de signal précédente,

- Détermination des fréquences des différentes séries de symboles,
- Détermination du un rapport de fréquence des séries de symboles corrélées par rapport aux séries anticorrélées dans une section du paramètre d'essai reproduit,
- Détermination de la différence relative des paramètres d'essai de deux sections adjacentes,
- Comparaison de la différence relative de deux paramètres d'essai avec une valeur seuil par défaut comme critère pour la stationnarité de la série temporelle du signal cardiologique représenté.

2. Appareil implantable selon la revendication 1, **caractérisé en ce que** la durée respective de l'intervalle RR du coeur est représentée comme signal cardiologique.

3. Appareil implantable selon l'une quelconque des revendications 1 ou 2, **caractérisé en ce que** la représentation des différentes valeurs de signal B(H) de la série temporelle est effectuée par une fonction de représentation

$$B_n (H) = 1 \text{ pour } B_{n+1} (H) - B_n (H) >/=0$$

et

$$B_n (H) = 0 \text{ pour } B_{n+1} (H) - B_n (H) < 0$$

sur des mots binaires d'une parité définie avec les éléments de symbole 0 et 1 comme séries de symboles respectives.

4. Appareil implantable selon la revendication 3, **caractérisé en ce que** les mots binaires contiennent respectivement cinq éléments de symbole.

5. Appareil implantable selon l'une quelconque des revendications 3 ou 4, **caractérisé en ce qu'**on détermine comme paramètre d'essai (M) la somme des fréquences de mots binaires ($P_{corr}$) avec les corrélations parallèles par rapport à la somme des fréquences de mots binaires ($P_{anticorr}$) avec des corrélations contraires selon le rapport

$$M = (\Sigma \, P_{corr})/(\Sigma \, P_{anticorr}).$$

6. Appareil implantable selon la revendication 5, **caractérisé en ce que** la différence relative ($\Delta$) des paramètres d'essai (M) de deux sections adjacentes est déterminée selon le rapport :

$$\Delta = (M_{i+1} - M_i)/M_i \times 100 \, \%$$

7. Appareil implantable selon la revendication 6, **caractérisé en ce que** la valeur seuil pour la stationnarité de la série temporelle s'élève à 50 %.

8. Appareil implantable selon l'une quelconque des revendications 1 à 7, **caractérisé en ce que** des sections connexes, dans lesquelles le critère de stationnarité est rempli, sont affectées comme base pour une nouvelle évaluation cardiologique des signaux cardiologique représentés.

FIG. 1

FIG. 2

FIG.3

FIG. 4

FIG. 5